# EUROPEAN PATENT SPECIFICATION

(11) **EP 1 488 801 B1**
(45) Date of publication and mention of the grant of the patent: **02.05.2007**
(21) Application number: 03744388.4
(22) Date of filing: 21.02.2003
(51) Int. Cl.: A61K 38/30, A61P 25/28

(54) **CHEMICAL IGF-I COMPOSITION FOR THE TREATMENT AND PREVENTION OF NEURODEGENERATIVE DISEASES**
CHEMISCHE IGF-I-ZUSAMMENSETZUNG ZUR BEHANDLUNG UND PRÄVENTION VON NEURODEGENERATIVEN ERKRANKUNGEN
COMPOSITION CHIMIQUE DE IGF-I UTILISEE DANS LE TRAITEMENT ET LA PREVENTION DE MALADIES NEURODEGENERATIVES

(30) Priority: 28.02.2002 ES 200200491
(43) Date of publication of application: 22.12.2004
(73) Proprietor: CONSEJO SUPERIOR DE INVESTIGACIONES CIENTIFICAS, 28006 Madrid (ES); UNIVERSIDAD COMPLUTENSE DE MADRID, 28040 Madrid (ES)
(72) Inventor: LOPEZ LOPEZ, C., Insto.Neurobiologia Ramon y Cajal, E-28002 Madrid (ES); CARRO DIAZ,E.M., Insto.Neurobiologia Ramon y Cajal, E-28002 Madrid (ES); TORRES ALEM N, I., Insto.Neurobiologia Ramon y Caj, E-28002 Madrid (ES); TORRADO DURAN, J. J., Univ.Complutense de Madrid, E-28040 Madrid (ES); TORRADO DURAN, S., Univ. Complutense de Madrid, E-28040 Madrid (ES); CARRASCOSA, Celia, Avda. De Séneca, 2, 28040 Madrid (ES)
(74) Representative: Carpintero Lopez, Francisco
(86) International application number: PCT/ES2003/000087
(87) International publication number: WO 2003/077940

(56) References cited:
- WO-A1-97/21442
- WO-A2-99/33481
- US-A- 5 093 317
- US-A- 5 633 228
- FERNANDEZ A.M. ET AL.: 'Neuroprotective action of peripherally administered insuline-like growth factor I in the injured olivo-cerebellar' RATHWAY EUROPEAN JOURNAL OF NEUROSCI. vol. II, 1999, pages 2019 - 2030, XP000984937

## Description

### FIELD OF THE INVENTION

The invention addresses the preparation and production of new formulations (microspheres and tablets) for the administration of active substances, including IGF-I, and their use in the treatment and prevention of neurodegenerative diseases like Alzheimer's disease or cerebellar ataxia, among others.

### BACKGROUND OF THE INVENTION

At present, there is no drug or method that protects against neurodegenerative diseases. This type of disease is growing in importance in developed countries due to progressive aging of the population because many such diseases are associated to age (Amaducci L, and Tesco G, Aging as a major risk for degenerative diseases of the central nervous system, *Curr Opin Neurol*, 7: 283-286 [1994]). Nevertheless, although the causes of diseases like Alzheimer's dementia or Parkinson's disease are being studied intensely and an important genetic component is beginning to be recognised (Heintz N, and Zoghbi HY, Insights from mouse models into the molecular basis of neurodegeneration, *Annu Rev Physiol,* 62: 779-802 [2000]), there is still no effective treatment, so prevention is extremely important. The fact that many of these diseases have a clear hereditary component makes it possible to predict the development, or predisposition to development, of a neurodegenerative disease. This means that protective measures can be taken against the disease before it appears. For example, a preventive measure that is on the verge of being tested in populations at risk of Alzheimer's disease is vaccination (Gurwitz D, Immunization for Alzheimer's disease: yet closer to clinical trials, *Trends Immunol.* 22: 542-543, [2001]) because there are subjects genetically predisposed to suffering the disease and advanced age is a risk factor.

A series of proteins of natural origin have therapeutic trophic hormonal activity and must be administered by the parenteral route because they are broken down by the digestive system. The best known of these proteins is insulin, and the search for alternative systems of administration has been going on for many years. Daily injections, even subcutaneous injections, have two main problems: the patient tends to avoid them (compliance declines), thus reducing bioavailability and, therefore, therapeutic efficacy. In the case of the trophic factor IGF-I, whose therapeutic efficacy in a wide range of diseases is currently being tested (for a list of clinical trials now under way in the United States: http://clinicaltrials.gov/ct/gui/c/a2b/action/GetStudy?JServSessionIdzone ct=1bfy4qa8h 1), it has been observed that repeated subcutaneous injections throughout the day are more effective than a single injection (Woodal SM, Breier BH, O'Sullivan U, Gluckman PD. The effect of the frequency of subcutaneous insulin-like growth factor I administration on weight gain in growth hormone deficient mice. *Horm Metab Res* 12: 581-584 [1991]), probably because the elevation in blood levels is more sustained.

One solution for this problem is being sought in the development of slow release systems (depot) for trophic factors, including IGF-I (Lam XM, Duenas ET, Daugherty AL, Levy N, and Cleland JL "Sustained release of recombinant human insulin-like growth factor-I for treatment of diabetes" *J Contr Rel* 67: 281-292 [2000]; Singh M, Shirley B, Bajwa K, Samara E, Hora M, and O'Hagan D, "Controlled release of recombinant insulin-like growth factor from a novel formulation of polylactide-co-glycolide microparticles" *J Contr Rel* 70: 21-28 [2001]; Meinel L, Illi OE, Zapf J, Malfanti M, Merkle HP, Gander B, "Stabilizing insulin-like growth factor-I in poly [D,L-lactide-co-glycolide] microspheres" *J Contr Rel* 70: 193-202 [2001]). Pharmaceutical companies specialised in trophic factors are working at developing such a formulation. Previous studies have demonstrated the usefulness of IGF-I formulated in controlled-release osmotic systems that are administered subcutaneously. These systems produce therapeutic effects in some pathologic processes (Fernandez AM, Gonzalez de la Vega A, Torres-Aleman I, "Insulin-like growth factor restores motor coordination in a rat model of cerebellar ataxia". *Proc Nat Acad Sci* (USA) 95: 1253-1258 [1998]). However, they have the disadvantage of being voluminous (and consequently, difficult to implant in humans) and requiring removal of the osmotic device when drug release ceases. In order to avoid this drawback, small, slow-release biodegradable preparations can be developed. Biodegradable lactic acid and glycolic acid copolymers, similar to those used in the manufacture of absorbable suture thread, are being used with increasing frequency for this purpose. There are different copolymers in which the proportion and viscosity of polylactic acid and polyglycolic acid can be varied. This makes it possible to control the rate of drug release and, consequently, to prolong drug absorption and the duration of its effects. Normally, the greater the proportion of polylactic acid in relation to polyglycolic acid, the slower the drug release rate is. On the other hand, the greater the intrinsic viscosity of the polymer, the slower the release rate is. Specifically, three studies of IGF-I have been published recently (Lam XM, Duenas ET, Daugherty AL, Levy N, and Cleland JL "Sustained release of recombinant human insulin-like growth factor-I for treatment of diabetes" *J Contr Rel* 67: 281-292 [2000]; Singh M, Shirley B, Bajwa K, Samara E, Hora M, and O'Hagan D, "Controlled release of recombinant insulin-like growth factor from a novel formulation of polylactide-co-glycolide microparticles" *J Contr Rel* 70: 21-28 [2001]; Meinel L, Illi OE, Zapf J, Malfanti M, Merkle HP, Gander B, "Stabilizing insulin-like growth factor-I in poly [D,L-lactide-co-glycolide] microspheres" *J Contr Rel* 70: 193-202 [2001]) in which a copolymer of 50:50 lactic acid and glycolic acid (Resomer 502H, Boehringer Ingelheim) was used. The preparation procedures involve either atomisation or the triple emulsion technique. Different systems are used in the triple emulsion technique to increase the size of the resulting microspheres. Thus, in the work of M. Singh et al. (Singh M, Shirley B, Bajwa K, Samara E, Hora M, and O'Hagan D, "Controlled release of recombinant insulin-like growth factor from a novel formulation of polylactide-co-glycolide microparticles" *J Contr Rel* 70: 21-28 [2001]), a change in pH is induced to produce partial precipitation of IGF-I, whereas in the work of L. Meinel et al. (Meinel L, Illi OE, Zapf J, Malfanti M, Merkle HP, Gander B, "Stabilizing insulin-like growth factor-I in poly [D,L-lactide-co-glycolide] microspheres" *J Contr Rel* 70: 193-202 [2001]), slow agitation speeds are used and proteins like gelatin and albumin are added to obtain microspheres of large size. In all these systems, the resulting microspheres are more than 30 micrometers in diameter, ranging from 30 to 125 micrometers depending on the study.

### Brief description

The invention addresses new therapeutic formulations for slow administration of IGF-I, a procedure for the preparation and production of these formulations, and their use in the manufacture of medicinal products for the treatment and prevention of neurodegenerative diseases like Alzheimer's disease or cerebellar ataxia, among others. These formulations correspond to microspheres less than 5 micrometers in diameter, among other characteristics, and to tablets for subcutaneous implantation.

### Detailed description

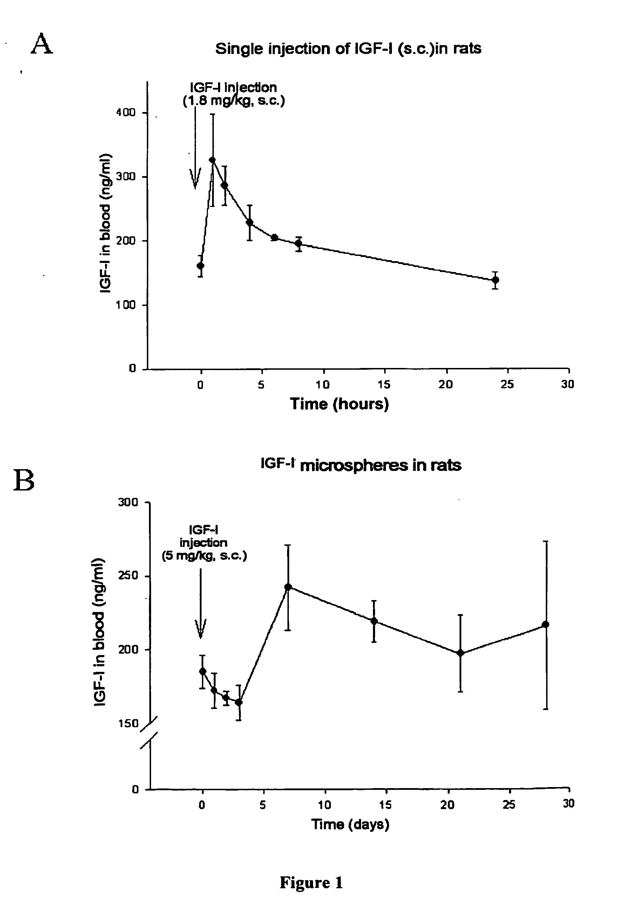

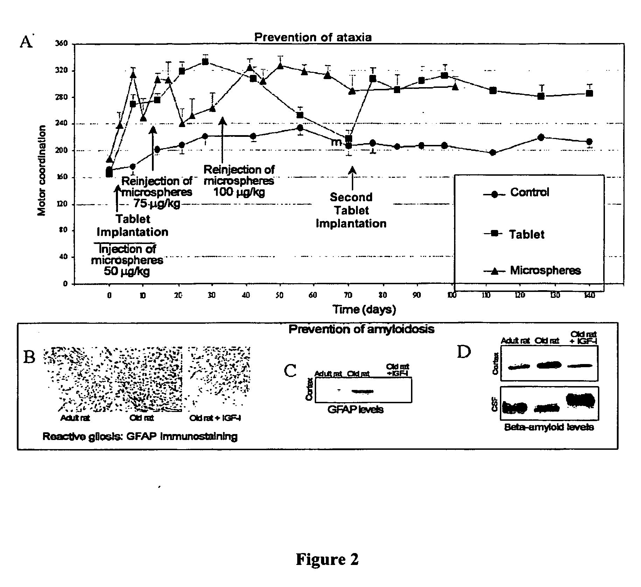

The invention is designed to prevent diseases of the nervous system caused by neuron loss of either genetic or sporadic origin. It consists of the subcutaneous administration of a long-lasting and biodegradable therapeutic formulation, which is developed in the present invention and contains the natural neuroprotective factor IGF-I. The novelty of this application is that it prevents the appearance of symptoms of neurodegenerative disease, as evaluated in animal models of hereditary cerebellar ataxia and Alzheimer's disease, and in old rats by periodic administration of this long-lasting formulation (see Examples 2, 3 and 4; Figure 2). In addition, administration of this formulation is very convenient (once or twice a month) (Figure 1B) and its therapeutic efficacy is greater than that of daily subcutaneous injections of IGF-I (Figure 1A) since therapeutic levels are more sustained. The route of administration is subcutaneous, so smaller particles (less than 5 micrometers) have been developed to pass more easily through injection needles.

Thus, one object of the present invention is a therapeutic formulation or composition that contains the natural neuroprotective factor IGF-I, herein referred to as the therapeutic formulation of the present invention, which allows the slow release of intact and functionally active IGF-I and is constituted by microspheres of high-viscosity copolymers less than 5 micrometers in diameter, generally between 1 and 2 micrometers, and preferably about 1.3 micrometers.

The therapeutic formulation proposed by this invention consists of a therapeutically effective amount of IGF-I together with a pharmaceutically suitable excipient. This therapeutic formulation is useful for administration and/or application in mammalian species, preferably human.

The therapeutically effective amount of the formulation of the present invention to be delivered, as well as the dosage of it for treating a pathologic state, depends on numerous factors, including age, patient state, disease severity, route and frequency of administration, and others.

The therapeutic formulation containing IGF-I provided by this invention can be delivered by any form of administration considered suitable for subcutaneous administration, including the necessary pharmaceutically acceptable excipients for formulation in accordance with the form of administration. A review of pharmaceutical forms for the administration of medicinal products and the necessary excipients can be found in the *Tratado de Farmacia Galenica* (Treaty of Galenic Pharmacy), C. Fauli i Trillo, 1993, Luzán 5, SA Editions, Madrid.

The possible effect on release rate has been compensated for by using a more viscous copolymer like Resomer 506 (Boehringer Ingelheim) instead of 502H as described in previous studies. Thus, the use of a copolymer with a viscosity of 0.8 dl/g is proposed, instead of the one described in the studies cited, which has a viscosity of 0.2 dl/g. To obtain smaller microspheres, the triple emulsion technique was used. In this technique a solution of IGF-I is used as the base, together with high homogenization rates to achieve the smallest possible internal phase in the emulsion. In addition, in the method proposed in the present invention, alkaline pH (at which IGF-I can become insoluble) and other high-molecular-weight proteins (albumin, gelatin, etc.) are not used. This yields small microspheres, generally 1-2 micrometers, which are more appropriate for subcutaneous administration by needle injection.

An additional object of the present invention is a procedure for the preparation and production of the therapeutic formulation referred to in the present invention, herein referred to as the procedure of the present invention, based on the triple emulsion technique and solvent extraction as follows:
- The copolymers used are of high viscosity, between 0.5 dl/g and 1.5 dl/g, preferably 0.8 dl/g,
- High homogenization rates are used, preferably 13500 rpm.
- The solutions used have a non-alkaline pH, and
- High-molecular-weight proteins, such as albumin and gelatin, are not used.

A particular object of the present invention is the procedure of the invention whereby the copolymer is 50:50 lactic acid and glycolic acid (PLGA 50:50), among others.

Another object of the present invention is the therapeutic formulation obtained with the procedure of the present invention.

As a reference and nonlimiting example of the triple emulsion technique, the published studies of Lam et al., Singh et al., and Meinel et al. are cited (Lam XM, Duenas ET, Daugherty AL, Levy N, and Cleland JL "Sustained release of recombinant human insulin-like growth factor-1 for treatment of diabetes" *J Contr Rel* 67: 281-292 [2000]; Singh M, Shirley B, Bajwa K, Samara E, Hora M, and O'Hagan D, "Controlled release of recombinant insulin-like growth factor from a novel formulation of polylactide-co-glycolide microparticles" *J Contr Rel* 70: 21-28 [2001]; Meinel L, Illi OE, Zapf J, Malfanti M, Merkle HP, Gander B, "Stabilizing insulin-like growth factor-I in poly [D,L-lactide-co-glycolide] microspheres" *J Contr Rel* 70: 193-202 [2001]).

On the other hand, as indicated in the introduction, these microspheres of lactic acid and glycolic acid copolymers can also be prepared by experts in the matter through the triple emulsion and solvent extraction procedure and through the atomisation technique, among others, which is why the therapeutic formulations of the present invention prepared and obtained using these techniques are part of the present invention. The atomisation procedure for the preparation of microspheres has been described in many studies, such as the following: P. Johansen, E. Estévez, R. Zurbriggen, H.P. Merkle, R. Gluck, G. Corradin, B. Gander. Towards clinical testing of a single-administration tetanus vaccines based on PLA/PLGA microspheres. *Vaccine* 2000. 19(9-10): 1047-54; P. Johansen, Y. Men, R. Audran, G. Corradin, H.P. Merkle, B. Gander. Improving stability of microencapsulated tetanus toxoid by co-encapsulation of additives. *Pharmaceutical Research* 1998. 15(7): 1103-10; S. Takada, Y. Uda, H. Toguchi, Y. Ogawa. Application of a spray-drying technique in the production of THR-containing injectable sustained-release microparticles of biodegradable polymers. *PDA Journal of Pharmaceutical Science and Technology* 1995. 49(4): 180-4; F. Pavanetto, I. Genta, P. Giunchedi, B. Conti. Evaluation of spray drying as a method for polylactide and polylactide-co-glycolide microsphere preparation. *Journal of Microencapsulation* 1993. 10(4):487-97; B. Bittner and T. Kissel. Ultrasonic atomization for spray drying: a versatile technique for the preparation of protein loaded biodegradable microspheres. *Journal of Microencapsulation* 1999. 16(3): 325-341.

Microspheres and tablets are both periodically administered by subcutaneous injection or implantation. The efficacy of protection against neurodegenerative disease is prolonged: Pcd mice, which suffer hereditary neurodegeneration, have been given this formulation for almost 5 months without developing hereditary neurodegenerative disease in that time. This means that while the drug is being administered, the disease does not appear, but it reappears if administration is interrupted. In the initial stages of the disease, re-administration of the product causes the symptoms of the disease to disappear (Figure 2A). On the other hand, administration of the therapeutic microsphere formulation to transgenic mice with Alzheimer's-like disease and to old rats reduced the symptoms associated with this disease, like beta-amyloid deposits and reactive gliotic lesions, among others (see Examples 3 and 4, Figure 2).

The present invention describes the use of any one of these formulations in the preparation of medicinal products for the treatment and prevention of neurodegenerative diseases like Alzheimer's disease, cerebellar ataxia, ataxia-telangiectasia, vascular dementia, multiple sclerosis, stroke, peripheral neuropathy, and cerebral or spinal trauma, among others. Thus, an object of the present invention is the use of the therapeutic formulation of the present invention in the preparation or production of medicinal products for the treatment and prevention of neurodegenerative diseases like Alzheimer's disease, cerebellar ataxia, ataxia-telangiectasia, vascular dementia, multiple sclerosis, stroke, peripheral neuropathy, and cerebral or spinal trauma, among others.

In addition to these small microspheres, tablets for subcutaneous implantation have been developed to obtain preparations with very long-lasting effects (see Example I c), herein referred to as tablets of the present invention, for which no references or data from previous studies by other authors have been found. Thus, an additional object of the present invention is this tablet for prolonged IGF-I release.

An additional object of the present invention is the procedure for the preparation and production of the tablet of the present invention, which is based on the following technique:
- IGF-I is dissolved in acetic acid 0.01 mM,
- Cyclosporin is added to this solution,
- This mixture is added to the lactic acid and glycolic acid copolymer (Resomer 503H of Boehringer Ingelheim) and gently kneaded, then the product is sieved through a 2-mm mesh,
- It is allowed to dry at room temperature for three hours and again sieved through 2-mm mesh, and
- It is allowed to dry another 15 hours at room temperature and compressed with concave punches, 6 mm in diameter

Finally, an additional object of the present invention is the use of the tablet of the present invention in the elaboration or preparation of medicinal products for the treatment and prevention of neurodegenerative diseases like Alzheimer's disease, cerebellar ataxia, ataxia-telangiectasia, vascular dementia, multiple sclerosis, stroke, peripheral neuropathy, and cerebral or spinal trauma, among others.

### DESCRIPTION OF DRAWINGS

**Figure 1.** IGF-I levels in blood after a single subcutaneous injection of IGF-I (**A**) remain high for less than 24 hours, whereas after subcutaneous injection (s.c.) of IGF-I microspheres (**B**), IGF-I levels stay high for at least 2 weeks.
**Figure 2.** Preventive effect of treatment with IGF-I depot in neurodegenerative diseases in **A)** mice with hereditary cerebellar ataxia treated with IGF-I microspheres (▲), IGF-I tablets (■), and an untreated control group (empty microspheres) (•). The degree of motor coordination is evaluated with the rota-rod test described in Fernandez et al. (1998). **B**) Old rats treated with IGF-I microspheres in which the amyloidosis associated to neurodegeneration during normal aging of the animals was evaluated by immunostaining of GFAP protein. **C**) Old rats treated with IGF-I microspheres in which amyloidosis associated to neurodegeneration during normal aging of the animals was evaluated by determining GFAP protein levels in brain (cortex). **D**) Old rats treated with IGF-I microspheres in which the amyloidosis associated to neurodegeneration during normal aging of the animals was evaluated by determining beta-amyloid levels in brain (cortex) and cerebrospinal fluid (CSF).

### EXAMPLES

### Example 1. Elaboration and administration of IGF-I preparations

### Example 1a. Subcutaneous injection of IGF-I (Figure 1A)

Lyophilised recombinant human IGF-I (rhIGF-I) (GroPep, Australia) is dissolved in saline solution (NaCl 0.9%) to obtain a concentration of 500 µg/100 µl. Adult Wistar rats weighing 300 grams (n=6 for each collection time) receive a single subcutaneous injection (in scapula) of 100 µl. To evaluate IGF-I in serum by radioimmunoassay (I. Torres-Aleman, S. Pons, L.M. Garcia-Segura. Climbing fiber deafferentation reduces insulin-like growth factor I (IGF-I) content in cerebellum. *Brain Res* 564: 348-351 [1991]; S Pons and I Torres-Aleman Basic fibroblast growth factor modulates insulin-like growth factor-I, its receptor, and its binding proteins in hypothalamic cell cultures. *Endocrinology* 131: 2271-2278 [1992]), blood is collected after anaesthesia and sacrifice of animals at different times after IGF-I injection.

As described in Figure 1A, a single subcutaneous injection of IGF-I (1.8 mg/kg) in adult rats caused a rapid increase in IGF-I levels, even though these levels persisted less than 24 hours (Figure 1A).

### Example 1b. Elaboration of IGF-I microspheres

The preparation of IGF-I microspheres was carried out using a modification of the triple emulsion and A/O/A solvent extraction method previously described (Singh M, Shirley B, Bajwa K, Samara E, Hora M, and O'Hagan D (2001) Controlled release of recombinant insulin-like growth factor from a novel formulation of polylactide-co-glycolide microparticles. *J*. *Control Release* 70: 21-28), which allows the effective release of intact and biologically active IGF-I (Meinel L, Illi OE, Zapf J, Malfanti M, Peter MH, and Gander B (2001) Stabilizing insulin-like growth factor-I in poly(D,L-lactide-co-glycolide) microspheres. *J. Control Release* 70: 193-202). The method begins with 40 mg of IGF-I dissolved in 100 microlitres of acetic acid 0.01 mM. To this dissolution is added 0.7 ml of monosodium phosphate 10 mM (pH 6.0) containing 10.5 mg of Tween 20 (Serva, Germany). At the same time, 500 mg of lactic acid and glycolic acid 50:50 copolymer (PLGA 50:50, Resomer^{®} 506, Germany), with an inherent viscosity of 0.8 dl/g, was dissolved in 10 ml of methylene chloride, which is mixed with the aqueous phase of IGF-I. The emulsion is homogenized at 13500 rpm using a Polytron^{®} for 2 minutes to obtain the first binary A/O emulsion of small particle size. Later, this emulsion is added to a solution of 400 ml of PBS buffer, pH 7.4, prepared for 1 litre with 6.8 g of monopotassium phosphate and 195.5 ml of NaOH 0.2 M solution. This PBS buffer contains 8 g of polyvinyl alcohol (polyvinyl alcohol 15000, Fluka, Switzerland). By pouring the first emulsion on the PBS solution and then shaking vigorously with a homogenizer at 13500 rpm for 2 minutes (Ultraturrax^{®}), a triple A/O/A emulsion is obtained. This triple emulsion is gently shaken (700 rpm) for 4 hours while the methylene chloride evaporates, thus forming microspheres. Later, the microspheres are recovered by centrifuging at 6000 rpm for 20 minutes and the supernatant is decanted. Next, microspheres are washed with deionised water and again centrifuged and decanted. The washing process is repeated three times. Finally, microspheres are lyophilised and refrigerated (4°C) until use.

The load of rhIGF-I in microspheres was determined by dissolution of 10 mg of microspheres in 1 ml of NaOH 1 N, which was shaken all night at room temperature. The protein concentration was evaluated by means of UV absorption at 284 nm using an extinction coefficient of 1043 (mg/ml)⁻¹cm⁻¹ for rhIGF-I in NaOH 1 N. Microsphere size was determined by laser diffraction (Galai^{®} Cis-I). The diluted samples of several batches were run in triplicate and the average diameter for each batch was considered. Microsphere morphology was analysed by means of electron microscopy (JEOL 6400). The resulting microspheres have a mean particle size of 1.3 ± 1 micrometers and a strength of 5%. The percentage of encapsulation was 70%.

Subcutaneous administration of these IGF-I microspheres to adult rats (5 mg/kg) produced IGF-I levels in blood that remained high for at least 2 weeks (Figure 1B). The relatively small diameter of these microspheres facilitated injection of this suspension through a 23G needle.

### Example 1c. Elaboration of IGF-I implantation tablets.

In order to prepare a batch of 10 tablets, 15 mg of IGF-I (GroPep, Australia) is weighed out and then dissolved in 1.5 ml of acetic acid 0.01 mM. Cyclosporin, 30 mg, is added to this solution and remains in suspension. This mixture is added to 455 mg of lactic acid and glycolic acid copolymer (Resomer 503H, Boehringer Ingelheim) and kneaded gently, and then the product is sieved through a 2-mm mesh. It is allowed to dry at room temperature for three hours and sieved again through 2-mm mesh. It is allowed to dry another 15 hours at room temperature and compressed with concave punches 6 mm in diameter, adjusting tablet weight to 50 mg. This yields biconvex tablets that are 6 mm in diameter, 3 mm thick, weigh 50 mg, and have an IGF-I content per tablet of 1.5 mg. These tablets are implanted by means of a small incision without anaesthesia underneath the skin of pcd mice; as biodegradable material, they do not need to be removed.

### Example 2. Treatment of mice with hereditary cerebellar degeneration (Purkinje cell degeneration) using IGF-I microspheres and implantation tablets.

Two-month-old mice with hereditary cerebellar ataxia (pcd, or "Purkinje cell degeneration", mice, Jackson Labs) that showed signs of the disease at one month of life (motor incoordination leading to premature death) were administered IGF-I microspheres by subcutaneous injection at a dose of 100 µg/kg once every two weeks after two first doses of 50 µg/kg and 75 µg/kg (Figure 2A). To determine whether symptoms remitted with treatment, motor capacity was evaluated once a week with the rota-rod test (A.M. Fernandez, A. Gonzalez de la Vega, I. Torres-Aleman. Insulin-like growth factor restores motor coordination in a rat model of cerebellar ataxia. *Proc Nat Acad Sci* (USA) 95: 1253-1258 [1998]). The experiment was carried out in two separate batches of 6 mice each time. The result was that mice showed a normal degree of coordination of movements while treatment (100 days) with either microspheres (Figure 2A, line with triangles) or tablets (Figure 2A, line with squares) continued in comparison with the findings in healthy mice (treated mice had maximum motor coordination values of almost 300 seconds, which was similar to the findings in healthy mice [data not shown]), whereas ataxia symptoms did not disappear in the control group (maximum motor coordination values of 200-240 seconds).

It should be noted that while a dose of IGF-I was being administered, whether by means of microspheres or tablets, the reduction in IGF-I levels correlated with the loss of motor coordination. When new doses of IGF-I were administered (after the second dose [14 days] and third dose [28 days] of microspheres and after the second tablet implantation [70 days]), disease symptoms again remitted. Note that the efficacy of treatment with microspheres depends on the dose administered (Figure 2A), in such a way that no loss of motor coordination is observed with the dose of 100 µg/kg, in contrast with the initial doses of 50 µg/kg and 75 µg/kg.

### Example 3. Treatment of transgenic mice with Alzheimer's-like disease using IGF-I microspheres.

In 10-month-old transgenic mice with excess beta-amyloid due to transgenesis of the mutated human APP gene (Tg2576 mice, see Hsiao et al., 1996 (Hsiao K, Chapman P, Nilsen S. Eckman C, Harigaya Y, Younkin S, Ynag F, Cole G. Correlative memory deficits, Abeta elevation, and amyloid plaques in transgenic mice. *Science* 274: 99-102, [1996]), kept under standard cage conditions: cycles of 12 hours of light and dark and water and food *ad libitum)* that were administered IGF-I microspheres for a month, cerebral beta-amyloid levels were evaluated by Western-blot. Beta-amyloid (βA) levels (as determined by evaluating the bands obtained by Western-blot with densitometry) in the cortex and hippocampus of untreated transgenic mice were 7 and 25 times higher than those found in disease-free littermates (hmutAPP^{-/-}), whereas transgenic mice treated with IGF-I had the same beta-amyloid levels as disease-free animals (p<0.01 versus untreated Tg2576).

### Example 4. Prevention of Alzheimer's disease (prevention of brain amyloidosis) in old rats with IGF-I microspheres

In old Wistar rats, 18 months-old, kept under standard light and food conditions, IGF-I microspheres were administered for 1 month and the appearance of gliotic lesions in cerebral parenchyma (frontal cortex) was determined by GFAP immunostaining (Lee, CK. Weindruch, R, Prolla, TA. Gene-expression profile of the ageing brain in mice. *Nature Genet* 25, 294-297 [2000]) (Figure 2b). Brain GFAP protein levels (Figure 2c) and beta-amyloid levels in brain (cortex) and cerebrospinal fluid (CSF) (Vaucher, E. et al. Amyloid β peptide levels and its effects on hippocampal acetylcholine release in aged, cognitively impaired and unimpaired rats. *J Chem Neuroanat* 21, 323-329 [2001]) (Figure 2d) were determined by Western-blot. High levels of GFAP protein or beta-amyloid peptide in brain and/or low levels of beta-amyloid peptide in cerebrospinal fluid are symptoms of amyloidosis associated to Alzheimer's neurodegeneration. All these parameters were similar between old rats treated with IGF-I and adult rats, whereas in the two latter cases levels were lower than those observed in untreated old rats (Figures 2b, 2c, and 2d). It should be noted that beta-amyloid levels in CSF were increased in old rats treated with IGF-I due to beta-amyloid clearance (Figure 2d, lower panel).

### Materials and methods

**Animals.** Inbred adult and old Wistar rats (250-450 g), transgenic Tg2576 mice, male and female pcd (Purkinje cell degeneration) mice, and litters of non-ataxic mice age and sex-matched with C57 mice as a control group were used in accordance with EU Directive 86/609/EEC. Animals were kept under standard laboratory conditions.
**Reagents**. 125 INa (Amersham, Great Britain) was used to iodinate rhIGF-I. Unless otherwise indicated, the rest of the reagents used in the present invention were from Sigma (USA) or Merck (Spain).
**Experimental design of IGF-I analysis in blood** : Multiple blood samples were taken from each adult rat and IGF-I levels were determined in serum before and after subcutaneous administration (s.c.) of rhIGF-I. Depending on the weight of the rats, the microsphere doses (50 µg/kg) were prepared as a suspension in sterile saline solution for immediate *bolus* injection. Another group of rats received a conventional subcutaneous *bolus* injection of a solution of 1.8 mg/kg of rhIGF-I. In every case blood samples were drawn from the subclavia at different times after injection. The relatively high doses used assured detection in serum after injection. Because it is difficult to make multiple extractions of blood samples in mice, samples were obtained from different mice at different times. Because pcd mice are difficult to breed, they were used only to assess the therapeutic efficacy of IGF-I microspheres.

To reduce interference with IGFBPs, serum samples were extracted using C₁₈ cartridges (Millipore, USA), whereas tissue samples were boiled in acetic acid 1 N for 30 minutes as has been described previously by inventors (Pons S and Torres-Aleman I (1992) Basic fibroblast growth factor modulates insulin-like growth factor-I, its receptor, and its binding proteins in hypothalamic cell cultures. *Endocrinology* 131: 2271-2278).

## Claims

1. Therapeutic formulation containing the natural neuroprotective factor IGF-I that allows slow release of intact and functionally active IGF-I, **characterised in that** it is constituted by microspheres of high-viscosity copolymers, 0.5 dl/g to 1.5 dl/g, with a diameter of less than 5 micrometers.

2. Therapeutic formulation according to claim 1, **characterised in that** the microspheres of copolymers have a viscosity of 0.8 dl/g and diameter of about 1.3 micrometers.

3. Procedure for the preparation and production of the therapeutic formulation according to claims 1 and 2 by means of the triple emulsion technique and solvent extraction, **characterised in that** it is carried out in the following way:
- The copolymers used are of high viscosity, between 0.5 dl/g and 1.5 dl/g, preferably 0.8 dl/g,
- High homogenization rates are used, preferably 13500 rpm,
- The solutions used have a non-alkaline pH, and
- High-molecular-weight proteins, such as albumin and gelatin, among others, are not used.

4. Procedure for the preparation and production of the therapeutic formulation according to claim 3, **characterised in that** the copolymer is, among others, a lactic acid and glycolic acid 50:50 copolymer (PLGA 50:50).

5. Use of the therapeutic formulation according to claims 1 and 2 in the elaboration or preparation of medicinal products for the treatment and prevention of neurodegenerative diseases like Alzheimer's disease, cerebellar ataxia, ataxia-telangiectasia, vascular dementia, multiple sclerosis, stroke, peripheral neuropathy, and cerebral or spinal trauma, among others.

6. Tablet with a capacity for prolonged release of IGF-I, **characterised in that** it is prepared by means of a method based on the following technique:
- IGF-I is dissolved in acetic acid 0.01 mM,
- Cyclosporin is added to this solution and remains in suspension,
- This mixture is added to the lactic acid and glycolic acid copolymer (Resomer 503H, Boehringer Ingelheim) and kneaded gently, then the product is sieved through a 2-mm mesh,
- It is allowed to dry at room temperature for three hours and sieved again through 2-mm mesh.
- It is allowed to dry another 15 hours at room temperature and compressed with concave punches 6 mm in diameter

7. Use of a tablet according to claim 6 in the elaboration or preparation of medicinal products for the treatment and prevention of neurodegenerative diseases like Alzheimer's disease, cerebellar ataxia, ataxia-telangiectasia, vascular dementia, multiple sclerosis, stroke, peripheral neuropathy, and cerebral or spinal trauma, among others.

## Patentansprüche

1. Therapeutische Zubereitung, enthaltend den natürlichen neuroprotektiven Faktor IGF-I, die eine langsame Freisetzung des intakten und funktionell wirksamen IGF-I erlaubt, **dadurch gekennzeichnet, dass** sie aus Mikrokugeln eines hochviskosen Copolymers, 0,5dl/g bis 1,5 dl/g, mit einem Durchmesser von weniger als 5 Mikrometer gebildet ist.

2. Therapeutische Zubereitung nach Anspruch 1, **dadurch gekennzeichnet, dass** die Mikrokugeln aus Copolymeren eine Viskosität von 0,5 dl/g und einen Durchmesser von etwa 1,3 Mikrometern aufweisen.

3. Verfahren zur Erzeugung und Herstellung der therapeutischen Zubereitung nach den Ansprüchen 1 und 2 nach der Dreiphasen-Emulsionstechnik und einer Lösungsmittelextration, **dadurch gekennzeichnet, dass** es in der folgenden Weise ausgeführt wird:
- die eingesetzten Copolymere sind solche einer hohen Viskosität zwischen 0,5 dl/g und 1,5 dl/g, vorzugsweise 0,8 dl/g,
- hohe Homogenisierungsgeschwindigkeiten werden verwendet, vorzugsweise 13500 UpM,
- die Lösungen haben einen nicht alkalischen pH-Wert und
- hochmolekulargewichtige Proteine wie Albumin und Gelatine u.a. werden nicht benutzt.

4. Verfahren zur Zubereitung und Herstellung der therapeutischen Zubereitung nach Anspruch 3, **dadurch gekennzeichnet, dass** das Copolymer unter anderem ein Milchsäure und Glykolsäure 50:50 Copolymer (PLGA 50:50) ist.

5. Verwendung der therapeutischen Zubereitung nach den Ansprüchen 1 und 2, in der Entwicklung oder Herstellung von Arzneimitteln zur Behandlung und Vorbeugung neurodegenerativer Krankheiten wie der Alzheimer Krankheit, der zerebellaren Ataxie, der Ataxia Teleangiectatica, der vaskulären Demenz, der Multiplen Sklerose, dem Schlaganfall, der peripheren Neuropathie und zerebraler oder spinaler Traumata u.a.

6. Tablette mit der Fähigkeit zur verlängerten Freisetzung von IGF-I, **dadurch gekennzeichnet, dass** sie hergestellt ist mittels einem Verfahren, dass auf der folgenden Technik beruht:
- IGF-I wird in 0,01 mM Essigsäure gelöst,
- Cyclosporin wird dieser Lösung zugesetzt und verbleibt suspendiert,
- dieses Gemisch wird dem Milchsäure und Glykolsäure Copolymer (Resomer 503H, Boehringer Ingelheim) zugesetzt und leicht verknetet, dann wird das Produkt durch ein Sieb einer Maschenweite von 2 mm gesiebt,
- es wird bei Raumtemperatur drei Stunden lang trocknen gelassen und erneut durch 2 mm Maschenweite gesiebt.
- Es wird weitere 15 Stunden lang bei Raumtemperatur trocknen gelassen und mit konkaven Stempeln eines Durchmessers von 6 mm verpresst.

7. Verwendung einer Tablette nach Anspruch 6, in der Entwicklung oder Herstellung eines Arzneimittels zur Behandlung und Vorbeugung neurodegenerativer Krankheiten wie der Alzheimer Krankheit, der zerebellaren Ataxie, der Ataxia Teleangiectatica, der vaskulären Demenz, der Multiplen Sklerose, dem Schlaganfall, der peripheren Neuropathie und zerebraler oder spinaler Traumata u.a.

## Revendications

1. Formulation thérapeutique contenant le facteur neuro-protecteur naturel IGF-I qui permet une libération lente de l'actif IGF-I de façon intacte et fonctionnelle, **caractérisée en ce qu'**il est constitué de microsphères de copolymères de viscosité élevée, 0,5 dl/g à 1,5 dl/g, avec un diamètre inférieur à 5 micromètres.

2. Formulation thérapeutique selon la revendication 1, **caractérisée en ce que** les microsphères de copolymères ont une viscosité de 0,8 dl/g et un diamètre d'environ 1,3 micromètres.

3. Procédure pour la préparation et la production de la formulation thérapeutique selon les revendications 1 et 2 aux moyens de la technique à triple émulsion et de l'extraction par solvant, **caractérisée en qu'**elle est réalisée de la manière suivante :
- les copolymères utilisés sont de viscosité élevée, entre 0,5 dl/g et 1,5 dl/g, de préférence 0,8 dl/g,
- de forts taux d'homogénéisation sont utilisés, de préférence 13500 rpm,
- les solutions utilisées ont un PH non alcalin, et
- des protéines à hauts poids moléculaire, telles que l'albumine et la gélatine, entre autres, ne sont pas utilisées.

4. Procédure pour la préparation et la production de le formulation thérapeutique selon la revendication 3, **caractérisée en ce que** le copolymère est, entre autre, un copolymère 50:50 d'acide lactique et d'acide glycolique (PLGA 50:50).

5. Utilisation de la formulation thérapeutique selon les revendications 1 et 2 dans l'élaboration ou la préparation de produits médicamenteux pour le traitement et la prévention des maladies neuro-dégénératives comme la maladie d'Alzheimer, l'ataxie cérébelleuse, l'ataxie télangiectasie, la démence vasculaire, la sclérose multiple, l'accident cérébro-vasculaire, la neuropathie périphérique, et les traumatismes cérébraux ou rachidiens, entre autres.

6. Comprimé avec une capacité de libération prolongée de l'IGF-I, **caractérisé en ce qu'**il est préparé au moyen d'une méthode basée sur la technique suivante :
- de l'IGF-I est dissout dans l'acide acétique 0,01 mM,
- de la cyclosporine est ajoutée à cette solution et reste en suspension,
- ce mélange est ajouté au copolymère d'acide lactique et d'acide glycolique (Résomer 503H, Boehringer Ingelheim) et malaxé doucement, puis le produit est tamisé à travers une maille de 2 mm,
- il est laissé sécher à température ambiante pendant trois heures et tamisé de nouveau à travers une maille de 2 mm,
- il est laissé sécher pendant encore 15 heures à température ambiante et comprimé avec des emporte-pièces concaves de 6 mm de diamètre.

7. Utilisation d'un comprimé selon la revendication 6 dans l'élaboration ou la préparation de produits médicamenteux pour le traitement et la prévention de maladies neuro-dégénératives comme la maladie d'Alzheimer, l'ataxie cérébelleuse, l'ataxie télangiectasie, la démence vasculaire, l'accident cérébro-vasculaire, la neuropathie périphérique, et les traumatismes cérébraux ou rachidiens, entre autres.
